# EUROPEAN PATENT SPECIFICATION

(11) **EP 0 965 268 B1**
(45) Date of publication and mention of the grant of the patent: **19.03.2003**
(21) Application number: 98670002.9
(22) Date of filing: 08.04.1998
(51) Int. Cl.: A01N 1/00, A01N 3/00, C07F 9/09, A61K 7/48

(54) **Thermostabilization, osmoprotection, and protection against desiccation of enzymes, cell components and cells by di-glycerol-phosphate**
Thermostabilisierung, Osmoseschütz und Trocknungsschütz von Enzymen, Zellinhaltstoffen und Zellen mit Di-glycerol-phosphat
Thermostabilisation, osmoprotection et protection contre la dessication d'enzymes, de la matière de cellules et de cellules avec di-glycérol-phosphate

(43) Date of publication of application: 22.12.1999
(73) Proprietor: bitop Aktiengesellschaft für biotechnische Optimierung, 58453 Witten (DE)
(72) Inventor: Santos, Helena, IBET and ITOB, 2780 Oeiras (PT); Lamosa, Pedro, IBET and ITOB, 2780 Oeiras (PT); Burke, Anthony, IBET and ITOB, 2780 Oeiras (PT); Maycock, Christopher, IBET and ITOB, 2780 Oeiras (PT)
(74) Representative: Thiel, Christian, Dr. Dipl.-Chem.

(56) References cited:
- EP-A- 0 354 474
- US-A- 3 975 545

## Description

The invention concerns the use of di-glycerol-phosphate for the stabilization of enzymes, other cellular components, and cells against general stress, namely caused by heat, high osmolarity, desiccation, freeze -drying, and repetitive use.

Several intracellular, small molecular solutes are known to accumulate in high levels when microorganisms are subjected to extreme environmental conditions, such as temperature or osmotic stress. These solutes generally serve as osmolytes in halotolerant and halophilic organisms.

Cells use osmolytes in order to maintain turgor pressure and cell volume under osmotic stress. Their intracellular accumulation to high levels allows normal enzyme activity, without damaging cellular structures. Thus, osmolytes are commonly designated as compatible solutes. In fact, compatible solutes are necessary for enzyme function in the dehydrated cytoplasm that exists under osmotic stress.

The extremely halophilic *Archaea* and a few halophilic anaerobic *Bacteria*, accumulate K⁺, Na⁺ and Cl⁻ in response to changes in the extracellular salt concentration, while all other microorganisms examined utilise organic compatible solutes for osmotic adjustment to water stress. The number of compatible solutes is relatively small, and fall into certain categories of compounds such as polyols and derivatives, sugars and derivatives, amino acids and amino acid derivatives, small peptides, betaines, and ectoines [1].

In addition to being compatible with the normal activities of the cell, osmolytes also seem to have a protective effect on the function of cell components, namely proteins. It has been shown that some compatible solutes are capable of stabilising proteins against temperature denaturation and denaturing agents such as detergents, organic solvents, urea and guanidine chloride [2, 3, 4].

The addition of small molecular weight solutes to solutions containing proteins leads to alterations in the physical properti es of water by increasing the surface tension and excluding solutes from the water -protein interface. In this way, the structure of water in the hydration shell of the protein becomes highly organised. This effect, reduces the entropy of the hydration shell, which is, by itself, thermodynamically unfavourable, and leads to the formation of a smaller protein-water interface. The opening to the solvent of protein side -chains, and the consequent rise in the water-protein interface area, is a common feature of the denaturation process. It is believed that the addition of compatible solutes to a protein solution reduces the exposed area of the protein, making the denaturation process more difficult and thereby, leading to a higher ratio of native to denatured forms.

The higher stability of proteins in the presence of certain small molecular weight organic solutes allows enzymes to function under more extreme conditions imposed by temperature, pressure, ionic strength, pH, detergents and organic solvents. One of the priorities of modern biotechnology is to obtain stable enzymes or agents that stabilise those enzymes against thermal or chemical denaturation. The ability of some compatible solutes to stabilise enzymes is, therefore, of the utmost importance to modern biotechnology.

It must also be stressed that compatible solutes protect proteins [5], cell membranes [6] and cells [7, 8] from the deleterious effects of desiccation. The preservation of desiccated or lyophilized cell components has many applications in medicine (conservation of tissues and other products of biological origin), in the pharmaceutical industry (microencapsulation of medicines), the cosmetic industry (liposomes), in the food industry (conservation of food), and scientific research (maintenance of cultures and cell lines). In spite of the great importance of desiccation and freezing in the conservation of biological samples, denaturation of proteins or a decrease of the viable count of cultures inevitably takes place during its use.

Dehydration of cell membranes and cells results in a phase transition of the lipid bilayer. After rehydration, the membranes undergo a reverse phase transition that can lead to the rupture of the membrane. The protection of membranes by low molecular weight solutes is related to the substitution of water between the polar head groups by these solutes, thereby maintaining the appropriate lipid phase when these structures are dehydrated.

Some thermophilic and hypertermophilic *Bacteria* and *Archaea* produce low molecular weight solutes. These solutes generally accumulate intracellularly during growth at supraoptimal temperatures and/or salinities leading to the suggestion that they exert a protective effect on, at least, some cell components. Under high growth temperatures, di*-myo*-inositol-(1,1')-phosphate and cyclic 2,3'-bisphosphoglycerate accumulate to high levels in *Pyrococcus furiosus and Methanothermus fervidus*, respectively [9, 10]. The novel compatible solute, β-mannosylglycerate, found in several thermophilic and hyperthermophilic *Bacteria* and *Archaea*, has recently been shown to have an important effect on the stabilisation of enzymes derived from mesophilic and thermophilic organisms [11].

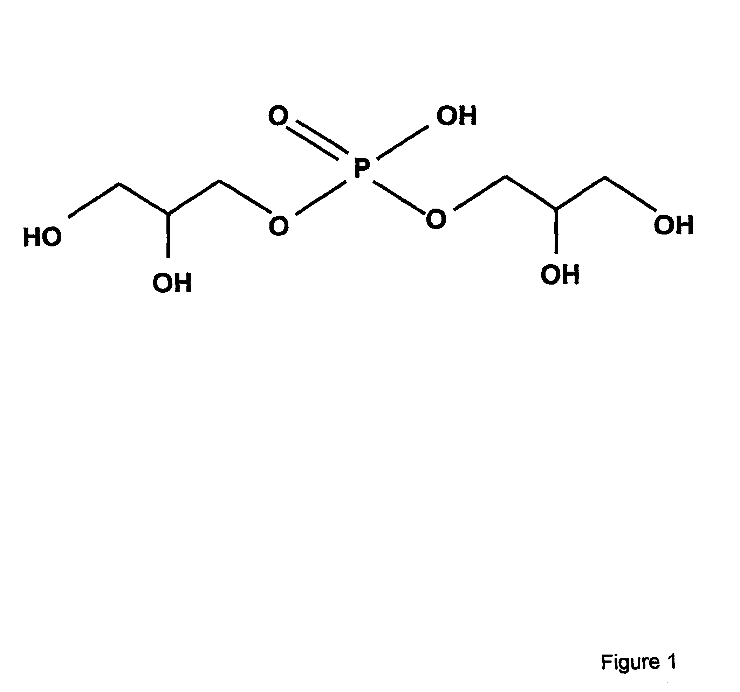

A novel compatible solute designated 1,1'-di-glycerol-phosphate (Figure 1) accumulates in the archaeon *Archaeoglobus fulgidus* [12]. Di-glicerol-phosphate represented in figure 1 can exist in all its stereoisomeric forms (racemic, meso and optically pure or mixtures thereof), and figure 1 is intended to include all such forms. In this species the intracellular concentration of this solute increases concomitantly with the salinity of the growth medium, and to a lesser extent at supraoptimal growth temperatures [12], indicating t hat 1,1'-di-glycerol-phosphate can be a thermostabilizer or osmoprotective agent.

Although, di-glycerol-phosphate had generically been previously envisioned as a possible cryopreservative in long term storage of tissues from animal or vegetable origin [EP-A-354474], its use as a highly efficient protector of enzymes, proteins, lipid vesicles or microbial cell lines against heat salt stress, desiccation or routine usage, had never been disclosed. 1,1' -di-glycerol-phosphate exerts highly stabilizing effects in the milimolar range concentration. This novel feature allows the use of this new solute as a protective agent of high efficiency at low concentration. As was pointed out above, conventional general protein stabilisers require high concentrations (in the molar range) to exert its effect, concentrations that in some cases may even hinder their use or make it not profitable.

### Figure 1.

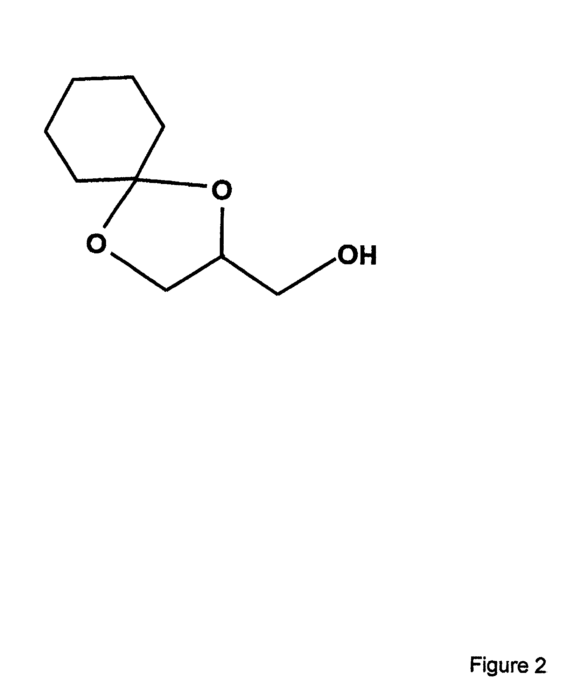

The chemical synthesis of 1,1'-di-glycerol-phosphate (Figure 1) can be accomplished readily and efficiently in three steps from commonly available glycerol in the following manner:
**Step1-** synthesis of 1,2-O-cyclohexylideneglycerol (Figure 2) according to Vogel's Textbook of Practical Organic Chemistry, p573 [13].

### Figure 2.

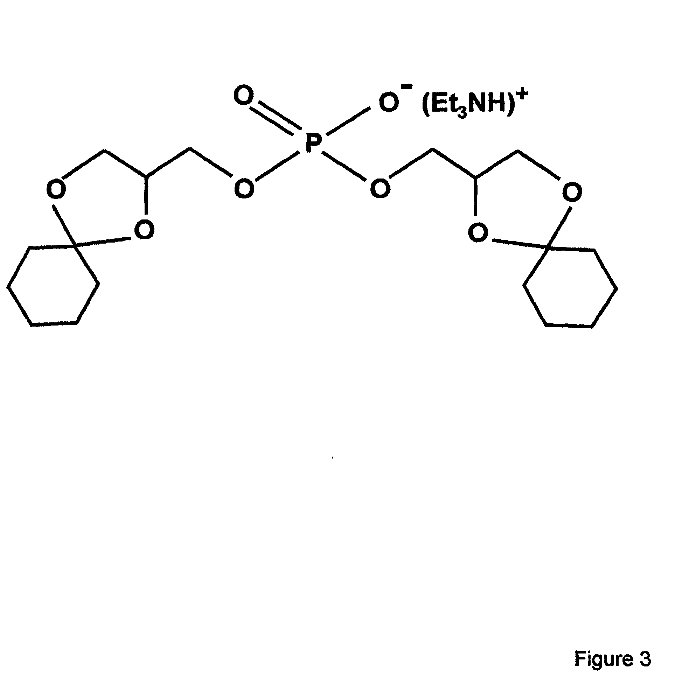

**Step2-** synthesis of 1,1'-bis(2,3-O-cyclohexylideneglycerol)phosphate triethylammonium salt (Figure 3) from the compound synthesized in step 1. This step is achieved by slowly adding 1,2-O-cyclohexylideneglycerol (prepared according to step 1) in triethylamine to a vigorously stirred solution of phosphoroyl chloride in anhydrous tetrahydrofuran at -78°C.

### Figure 3.

**Step3-** synthesis of 1,1'-di-glycerol-phosphate from the compound prepared in step 2. This step is achieved by the overnight treatment, at room temperature, of 1,1'-bis(2,3 -O-cyclohexylideneglycerol)phosphate triethylammonium salt with activated Dowex 50W-X8 (H⁺) ion exchange resin in order to remove the acetal protecting groups and for the isolation of the free 1,1'-di-glycerol-phosphate as the free acid.

Also, stereospecific synthesis of (S,S)-1,1'-di-glycerol-phosphate can be accomplished from commonly available D-mannitol in four steps.

In the first two steps (S)-1,2-O-isopropylideneglycerol is obtained by the reduction of the corresponding aldehyde (prepared from D-mannitol by the procedures delineated on pgs 654 and 592 of Vogel's Textbook of Practical Organic Chemistry, [13]), according to the procedure of M.E. Jung and T.J. Shaw [14]. As third and fourth steps (S)-1,2-O-isopropylideneglycerol is converted, through the intermediacy of (S,S)-1,1'-bis(2,3-O-isopropylideneglycerol)phosphate triethylammonium salt to (S,S)-1,1'-di-glycerol-phosphate, in exactly the same manner as described for the racemic compound.

1-1'-di-glycerol-phosphate is considerably better than glycerol as a thermoprotectant for enzymes. This product can be used as a stabilising agent of several biological systems, such as, proteins, enzymes, biological or artificial membranes or cells, vulnerable to damage and decreased efficiency (or viability) under harsh or stressing conditions, such as high temperatures, high salt concentrations, desiccation and/or freezing, and the action of denaturing agents (organic solvents, detergents, oxidizing agents and chaotropic substances). In addition, 1,1'-di-glycerol-phosphate can be used as an additive in cosmetics, either for stabilisation of liposomes or for the improvement of moisturising properties.

### BRIEF DESCRIPTION OF THE DRAWINGS

**Figure 1** depicts the chemical structure of 1,1'-di-glycerol-phosphate in all its stereoisomeric forms.

**Figure 2** depicts the chemical structure of 1,2-O-cyclohexylideneglycerol.

**Figure 3** depicts the chemical structure of 1,1'-bis(2,3-O-cyclohexylideneglycerol)phosphate (triethylammonium salt).

**Figure 4** shows the effect of 1-1'-di-glycerol-phosphate on the thermostability of *Desulfovibrio gigas* rubredoxin. The percentage of native protein after 30 minutes at 90°C as judged from the measurement of absorbance at 494 nm in:
**A:** absence of solutes, **B:** 100 mM potassium phosphate, **C:** 200 mM glycerol, and **D:** 100 mM 1,1'-di-glyderol-phosphate, is presented.

**Figure 5** shows the effect of di-glycerol-phosphate on enzymic activities after 10 min incubation at 50°C of rabbit muscle lactate dehydrogenase (A) and baker's yeast alcohol dehydrogenase (B); W: absence of solute; G: in the precence of 100mM 1,1'-di-glycerol-phosphate.

**Figure 6** shows the effect of the concentration of 1,1'-di-glycerol-phosphate (●) on the thermal stability of rabbit muscle lactate dehydrogenase after 10 min incubation at 50°C.

### EXAMPLE 1

This example shows that 1,1'-di-glycerol-phosphate (Figure 1) can be synthesised from 1,1'-bis(2,3-O-cyclohexylideneglycerol)phosphate triethylammonium.

As a first step synthesis of 1,1'-bis(2,3-O-cyclohexylideneglycerol)phosphate triethylammonium salt was accomplis hed by the following manner: 1,2-O-cyclohexylideneglycerol (2.0 g, 11.6 mmol) (prepared according to [13] p573), in triethylamine (10 mL, 71.2 mmol) was added slowly to a vigorously stirred solution of phosphoroyl chloride (0.56 mL, 6.0 mmol) in anhydrous tetrahydrofuran (10 mL) at -78°C. Upon complete addition of the substrate/triethylamine the reaction mixture was allowed to stir at -78°C for 10 minutes before being treated with water (10 mL) and then warmed to room temperature. Stirring was continued for about 10 minutes before the organic phase was separated, and the aqueous phase extracted twice with ethyl acetate. The aqueous phase was evaporated to dryness giving a white solid that was dissolved in approximately 20 mL dichloromethane. Exhaustive treatment of this solution with diethyl ether allowed precipitation of the triethylammonium hydrochloride byproduct and eventually gave only the title compound as a colourless gum (1.458 g, 25%).

As a second step, 1,1'-bis(2,3-O-cyclohexylideneglycerol)phosphate triethylammonium salt (1.458 g, 2.87 mmol) dissolved in distilled water was treated with about 30 g of activated Dowex 50W-X8 ion exchange resin. The heterogeneous mixture was stirred vigorously overnight, at room temperature, after which the ion exchange resin was filtered, washed with distilled water and the filtrate evaporated to dryness to furnish the 1,1'-di-glycerol-phosphate (0.765 g, 85%).

### EXAMPLE 2

This example shows that (S,S)-1,1'-di-glycerol-phosphate can be synthesised from D-mannitol in four steps.

In step 1 the (S)-1,2-O-isopropylideneglyceraldehyde (0.425 g, 3.22 mmol) was prepared from D-mannitol by the procedures delineated on pgs 654 and 592 of Vogel's Textbook of Practical Organic Chemistry, [13]

Step 2 consisted in the preparation of (S)-1,2-O-isopropylideneglycerol by the reduction of the corresponding aldehyde, synthesised in step 1, according to the procedure of M. E. Jung and T. J. Shaw [14].

In step 3 (S)-1,2-O-isopropylideneglycerol was converted into (S,S)-1,1'-bis(2,3-O-isopropylideneglycerol)phosphate triethylammonium in exactly the same manner as 1,1'-bis(2,3-O-cyclohexylideneglycerol)phosphate triethylammonium of example 1 was prepared from 1,2-O-cyclohexylideneglycerol.

As a final step (S,S)-1,1'-di-glycerol-phosphate (0.390 g, 49%), was obtained by the overnight treatment, at room temperature, of 1,1'-bis(2,3-O-isopropylideneglycerol)phosphate triethylammonium salt with about 20 g of activated Dowex 50W-X8 ion exchange resin. The heterogeneous mixture was stirred vigorously, after which the ion exchange resin was filtered, washed with distilled water and the filtrate evaporated to dryness to furnish the (S,S) -1,1'-di-glycerol-phosphate.

### EXAMPLE 3

This example shows that 1,1'-di-glycerol-phosphate has a thermostabilising effect on protein structures using *Desulfovibrio gigas* rubredoxin as a model-system.

Rubredoxin was obtained and purified as described by LeGall and Dragoni [15] from *Desulfovibrio gigas.* This protein was then stored at -20°C in 10 mM Tris-HCl buffer at pH 7.6.

The most informative feature of the UV-Vis absorption spectrum of rubredoxins comes from the signals with maxima centered at 380, 494, and 570 nm which are attributed to metal to ligand transitions. These bands are bleached when rubredoxin undergoes de naturation.

In the experiments designed to evaluate denaturation caused by thermal stress, rubredoxin was prepared at a concentration of 84 µg . ml⁻¹ in 10 mM Tris-HCI buffer pH 7.6 and the incubation mixtures, with or without the additions of solutes, were placed in 400 µl quartz cells in a Beckman spectrophotometer with a thermostated cell compartment. The protein was subjected to a temperature of 90°C and the decrease in the absorbance at 494 nm was monitored as a function of time.

### Figure 4.

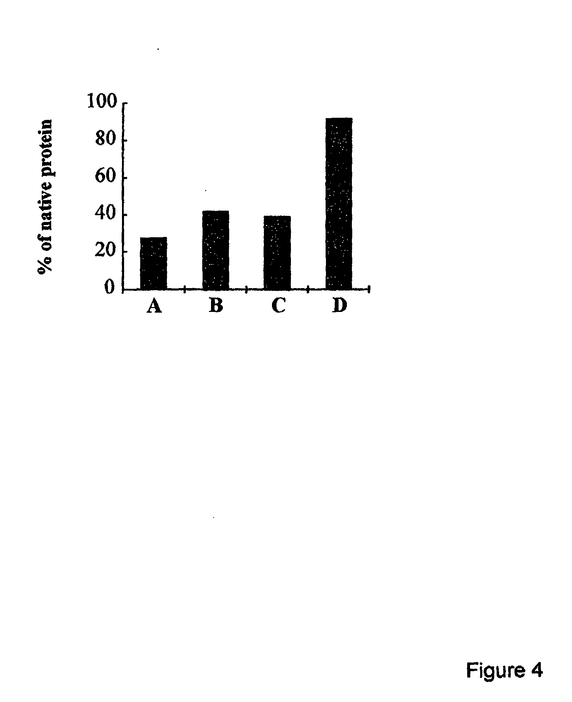

The protective effect of 1,1'-di-glycerol-phosphate (at a concentration of 100 mM) was compared with that of potassium phosphate (100 mM) and glycerol (200 mM) (Figure 4). After 30 min incubation of rubredoxin at 90 °C the absorbance decreased by only 8.5% when 1,1'-di-glycerol-phosphate was present, whereas in the absence of any solute a decrease of 73% was observed. Furthermore, a comparison of the effect exerted by 1,1'-di-glycerol-phosphate with that of glycerol or potassium phosphate, clearly showed that these so lutes were not as efficient as 1,1'-di-glycerol-phosphate. In fact, the decreases in absorption are as high as 61 and 58 % in the presence of 200 mM glycerol or 100 mM potassium phosphate, respectively. This demonstrates the strong thermoprotective effect exerted by 1,1'-di-glycerol-phosphate on rubredoxin.

### EXAMPLE 4

This example shows that 1,1'-di-glycerol-phosphate has a thermoprotective effect on the activity of enzymes used as model -systems, namely rabbit muscle lactate dehydrogenase (LDH) and yeast alcohol dehydrogenase (ADH), and can be used to protect enzymes in general against thermal deactivation.

Lactate dehydrogenase, LDH (Sigma Type III, rabbit muscle), purchased as a suspension in ammonium sulphate, was centrifuged, the supernatant was discarded, and the enzyme was suspended in 50 mM potassium phosphate pH 7.5. Alcohol dehydrogenase (Sigma, baker's yeast) was obtained in the lyophilized form and used without further purification. Enzymes were assayed using a Beckman spectrophotometer with a the rmostated cell compartment at 30°C.

In the experiments where thermal stress was imposed, enzymes were prepared at a concentration of 50 mg . ml⁻¹ in 20 mM potassium phosphate buffer, and the reaction mixtures were placed in Eppendorf tubes, incubated in a water-bath at 50°C for 10 minutes, cooled in an ice-bath and assayed immediately under standard conditions.

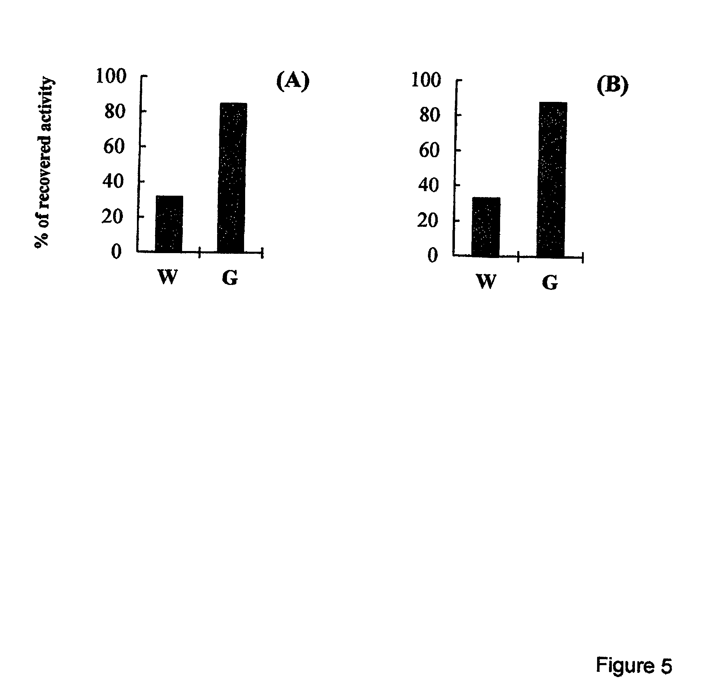

The effect of 1,1'-di-glycerol-phosphate on the thermostability of rabbit muscle LDH and baker's yeast ADH was compared to that of glycerol, a compound that is generally described as an enzyme protector. The percentage of residual activity in relation to pre-incubation activity, measured after 10 min of incubation at 50°C, is shown in Figure 5, for rabbit muscle LDH (A) and yeast ADH (B).

### Figure 5.

1,1'-di-glycerol-phosphate exerted a protective effect on lactate dehydrogenase (LDH). In fact, after 10 min of incubation at 50°C, LDH retained 85% activity in the presence of 100 mM of 1,1'-di-glycerol-phosphate whereas in the presence of 100 mM glycerol or in the absence of any solute, only 37% or 30% of residual activity was recovered, respectively. Alcohol dehydrogenase showed a similar behaviour. The activity retained after 10 min incubation at a temperature of 50°C was only of 33% of the initial ac tivity in the absence of any solute whereas in the presence of 100 mM 1,1' -di-glycerol-phosphate that value was as high as 88%, and in 200 mM glycerol it was 31%.

This comparison between the effects exerted by glycerol and 1,1'-di-glycerol-phosphate, showed that 1,1'-di-glycerol-phosphate is a much more efficient thermoprotector of LDH and ADH than glycerol.

### Figure 6.

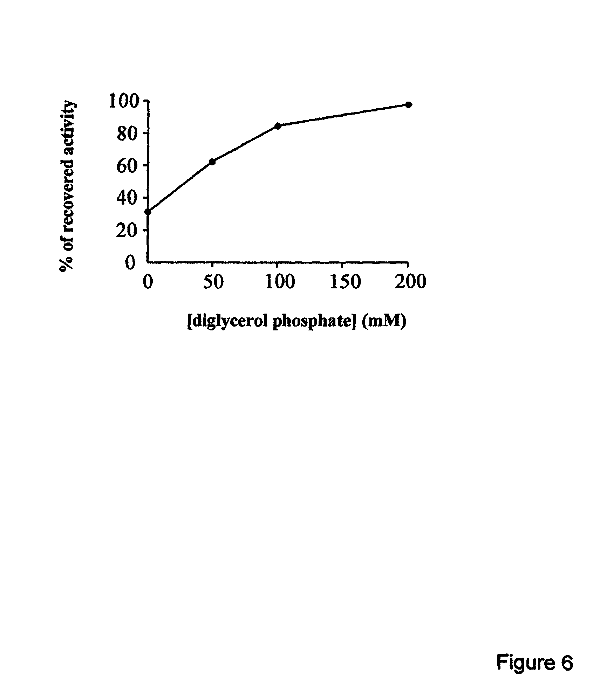

The effect of the concentration of 1,1'-di-glycerol-phosphate on the thermal stability of LDH was also monitored, and the results obtained after 10 min incubation at 50°C are shown in Figure 6. Increased concentrations of 1,1'-di-glycerol-phosphate were increasingly effective on protecting LDH against thermal stress. Concentrations of 1,1'-di-glycerol-phosphate higher than 200 mM were not tested. However, the increase in protection was much more pronounced when the concentration of 1,1'-di-glycerol-phosphate was increased from 50 to 100 mM, when compared to the effect when the concentration was further increased to 200 mM; this suggests "satur ation" of the effect on LDH for a concentration of 1,1'-di-glycerol-phosphate in the range 100 to 200 mM.

### REFERENCES:

**[1]** Brown, A. D.. (1990). Microbial Water Stress Physiology - Principles and Perspectives. John Wiley & Sons, Chichester, New York, Brisbane, Toronto, Singapore.
**[2]** Arakawa, T. and S. N. Timasheff. (1985). The stabilization of proteins by osmolytes. Biophys. J.. **47:** 411-414.
**[3]** Arakawa, T. and S. N. Timasheff, (1983). Preferential interactions of proteins with solvent components in aqueous amino acid solutions. Arch. Biochem. Biophys.. **224:** 169-177.
**[4]** Arakawa, T. and S. N. Timasheff. (1982). The stabilization of protein structure by sugars. Biochem.. **21:** 6536-6544.
**[5]** Carpenter, J. F., Crowe, J. H. and T. Arakawa. (1990). Comparison of solute-induced protein stabilization in aqueous solution and in the frozen and dried states. J. Dairy Sci.. **73**: 3627-3636.
**[6]** Rudolph, A. S., Crowe, J. H. and L. M. Crowe. (1986). Effects of three stabilizing agents - proline, betaine, and trehalose - on membrane phospholipids. Arch. Biochem. Biophys.. **245:** 134-143.
**[7]** Louis, P., Truper, H. G. and E. A. Galinski. (1994). Survival of *Escherichia coli* during drying and storage in the presence of compatible solutes. Appl. Microbiol. Biotechnol.. **41**: 684-688.
**[8]** Leslie, S. B., Teter, S. A., Crowe, L. M. and J. H. Crowe, (1994). Trehalose lowers membrane phase transitions in dry yeast cells. Biochm. Biophys. Acta. 1192: 7-13.
**[9]** Martins, L. O. and H Santos. (1995). Accumulation of mannosylglycerate and di-*myo*-inositol-phosphate by *Pyrococcus furiosus* in response to salinity and temperature. Appl. Environ. Microbiol.. **61:** 3299-3303.
**[10]** Hensel, R. and H. Konig. (1988). Thermoadaptation of methanogenic bacteria by intracellular ion concentration. FEMS Microbiol. Lett.. **49**: 75-79.
**[11]** Ramos, A., N. D. H. Raven, R. J. Sharp, S. Bartolucci, M. Rossi, R. Cannio, J. Lebbink, J. van der Oost, W. M. de Vos and H. Santos. 1997. Stabilization of enzymes against thermal stress and freeze-drying by mannosylglycerate. Appl. Environ. Microbiol.. **63**: 4020-4025.
**[12]** Martins, L. O., R. Huber, H. Huber, K. O. Stetter, M. S. da Costa and H. Santos. 1997. Organic solutes in hyperthermophilic *Archaea.* Appl. Environ. Microbiol.. **63:** 896-902.
**[13]** Vogel, A. I.. Vogel's Textbook of Practical Organic Chemistry. Fifth edition, 1989. Rev. by B. S. Furniss, A. G. Hannaford, P. W. G. Smith, and A. R. Tatchell. Longman Scientific Technical. Burnt Mill, Harlow, Essex, England.
**[14]** Jung, M. E. and T. J. Shaw. (1980). Total synthesis of (R)-glycerol acetonide and the antiepileptic and hypotensive drug (-)-γ-amino-β-hydroxybutyric acid (GABOB): use of vitamine C as a chiral starting material. J. Amer. Chem. Soc., **102:** 6304-6311.
**[15]** J. LeGall and N. Dragoni (1966). Dependence of sulfite-reduction on a crystallized ferredoxin from *Dessulfovibrio gigas..* Biochem. Biophys. Res. Comm. **23:** 145-149.

**[EP-A-354474]** Glonek, T., and J. V. Greiner. 1989. Method and composition for cryopreservation of tissue. European Patent Application n° 89114343.0.

## Claims

1. The utilization of 1,1'-di-glycerol-phosphate wherein the compound is in any of its possible stereoisomeric forms, specifically in its racemic, meso and/or optically active forms, as a stabilizer of cell components or cells against stress.

2. Chemical synthetic process of 1,1'-di-glycerol-phosphate represented in figure 1 wherein the compound is synthesized in three steps from commercially available glycerol. As a first step 1,2 -O-cyclohexylideneglycerol is prepared. As a second step 1,1' -bis(2,3-O-cyclohexylideneglycerol)phosphate triethylammonium salt is prepared by the reaction of 1,2-O-cyclohexylideneglycerol with phosphoroyl chloride in anhydrous tetrahydrofuran. As a third step 1,1'-di-glycerol-phosphate is synthesized by treating 1,1'-bis(2,3-O-cyclohexylideneglycerol)phosphate triethylammonium salt with activated Dowex 50W-X8 ion exchange resin.

3. Chemical synthetic process of optically pure (S,S)-1,1'-di-glycerol-phosphate wherein the compound is synthesized from commercially avail able D-mannitol. In the first two steps (S)-1,2-O-isopropylideneglycerol is obtained by the reduction of the corresponding aldehyde. In the third and fourth steps (S)-1,2-O-isopropylideneglycerol is converted, through the intermediacy of (S,S)-1,1'-bis(2,3-O-isopropylideneglycerol)phosphate triethylammonium into (S,S)-1,1'-di-glycerol-phosphate, according to claim 2.

4. Chemical synthetic process of optically pure (R,R)-1,1'-di-glycerol-phosphate wherein the compound is synthesized from commercially availa ble D-mannitol. In the first two steps (R)-1,2-O-isopropylideneglycerol is obtained by the reduction of the corresponding aldehyde. In the third and fourth steps (R)-1,2-O-isopropylideneglycerol is converted, through the intermediacy of (R,R)-1,1'-bis(2,3-O-isopropylideneglycerol)phosphate triethylammonium salt into (R,R)-1,1'-di-glycerol-phosphate, according to claim 2.

5. Chemical synthetic process of 1,1'-di-glycerol-phosphate according to claims 2, 3 and 4 wherein the compound is in any of its possible stereoisomeric forms, specifically in its racemic, meso and/or optically active forms.

6. The utilization of 1,1'-di-glycerol-phosphate according to claim 1 wherein as thermostabilizing agent on lactate dehydrogenase, alcohol dehydrogenase, and rubredoxin, and any other enzyme of microbial, plant or animal sources to improve its thermal stability.

7. The utilization of 1,1'-di-glycerol-phosphate according to claim 1 wherein to protect enzymes or other proteins against temperature denaturation induced by purification, transport and/or storage.

8. The utilization of 1,1'-di-glycerol-phosphate according to claim 1 wherein as a protector of the activity of polymerase chain reaction (PCR) enzymes for clinical, biological and industrial purposes during the s torage, as well as the high-temperature recycling of the enzymes.

9. The utilization of 1,1'-di-glycerol-phosphate according to claim 1 wherein as a stabiliser of enzymes or other proteins during lyophilization, desiccation or freeze-drying and storage at low temperatures.

10. The utilization of 1,1'-di-glycerol-phosphate according to claim 1 wherein as a stabilising agent during the manufacture, storage and assays using test kit enzymes, thereby prolonging storage and higher efficiency of said enzymatic test kits for diagnostic, biological and industrial purposes.

11. The utilization of 1,1'-di-glycerol-phosphate according to claim 1 wherein to stabilise enzymes or other proteins during their routine utilization for clinical, biological and industrial purposes, namely when used in biosensors.

12. The utilization of 1,1'-di-glycerol-phosphate according to claim 1 wherein for the protection against stress such as desiccation and lyophilization of cell membranes, liposomes, liposome -containing cosmetics or lipid related substances.

13. The utilization of 1,1'-di-glycerol-phosphate according to claim 1 wherein as an additive to cosmetics.

14. The utilization of 1,1'-di-glycerol-phosphate according to claim 1 wherein for protection against damage caused by lyophilization, desiccation, high temperatures, and freezing to microbial cells.

## Patentansprüche

1. Verwendung von 1,1'-Diglycerol-phosphat, wobei die Verbindung in einer ihrer möglichen stereoisomeren Formen vorliegt, insbesondere als Racemat, in der meso- und/oder einer optisch aktiven Form, als Stabilisator für Zellkomponenten oder Zellen bei Belastung.

2. Verfahren zur chemischen Synthese von 1,1'-Diglycerol-phosphat, dargestellt in Figur 1, wobei die Verbindung, ausgehend von kommerziell erhältlichem Glycerol, in 3 Stufen synthetisiert wird. In einem ersten Schritt wird 1,2-O-Cyclohexylidenglycerol hergestellt. In einem zweiten Schritt wird 1,1'-Bis-(2,3-O-Cyclohexylidenglycerol)-phosphat Triethylammoniumsalz durch Reaktion von 1,2-O-Cyclohexylidenglycerol mit Phosphorylchlorid in wasserfreiem Tetrahydrofuran hergestellt. In einem dritten Schritt wird 1,1'-Diglycerol-phosphat durch Behandlung von 1,1'-Bis-(2,3-O-Cyclohexylidenglycerol)-phosphat Triethylammoniumsalz mit aktiviertem Dowex 50W-X8 lonenaustauscherharz synthetisiert.

3. Verfahren zur chemischen Synthese von optisch reinem (S,S)-1,1'-Diglycerol-phosphat, wobei die Verbindung aus kommerziell erhältlichem D-Mannit synthetisiert wird. In den ersten beiden Schritten wird (S)-1,2-O-Isopropylidenglycerol durch Reduktion des entsprechenden Aldehyds erhalten. Im dritten und vierten Schritt wird (S)-1,2-O-lsopropylidenglycerol über die Zwischenstufe des (S,S)-1,1'-Bis-(2,3-O-Isopropylidenglycerol)-phosphat Triethylammoniumsalzes in (S,S)-1,1'-Diglycerol-phosphat umgewandelt, entsprechend Anspruch 2.

4. Verfahren zur chemischen Synthese von optisch reinem (R,R)-1,1'-Diglycerol-phosphat, wobei die Verbindung aus kommerziell erhältlichem D-Mannit synthetisiert wird. In den ersten beiden Schritten wird (R)-1,2-O-Isopropylidenglycerol durch Reduktion des entsprechenden Aldehyds erhalten. Im dritten und vierten Schritt wird (R)-1,2-O-Isopropylidenglycerol über die Zwischenstufe des (R,R)-1,1'-Bis-(2,3-O-Isopropylidenglycerol)-phosphat Triethylammoniumsalzes in (R,R)-1,1'-Diglycerol-phosphat umgewandelt, entsprechend Anspruch 2.

5. Verfahren zur chemischen Synthese von 1,1'-Diglycerol-phosphat nach den Ansprüchen 2, 3 und 4, wobei die Verbindung in einer ihrer möglichen stereoisomeren Formen vorliegt, insbesondere als Racemat, als meso- und/oder optisch aktive Form.

6. Verwendung von 1,1'-Diglycerol-phosphat nach Anspruch 1 als thermostabilisierender Stoff für Lactatdehydrogenase, Alkoholdehydrogenase, Rubredoxin und andere Enzyme mikrobiellen, pflanzlichen oder tierischen Ursprungs zur Verbesserung ihrer Hitzestabilität.

7. Verwendung von 1,1'-Diglycerol-phosphat nach Anspruch 1 zum Schutz von Enzymen oder anderen Proteinen gegen Temperaturdenaturierung, hervorgerufen durch Reinigung, Transport und/oder Lagerung.

8. Verwendung von 1,1'-Diglycerol-phosphat nach Anspruch 1 als Schutz der Aktivität von Enzymen für die Polymerase-Kettenreaktion (PCR) für klinische, biologische und industrielle Zwecke während der Lagerung der Enzyme als auch während der Hochtemperaturzyklen.

9. Verwendung von 1,1'-Diglycerol-phosphat nach Anspruch 1 als Stabilisator von Enzymen oder anderen Proteinen während der Lyophilisierung, Trocknung oder Gefriertrocknung und Lagerung bei niedrigen Temperaturen.

10. Verwendung von 1,1'-Diglycerol-phosphat nach Anspruch 1 als Stabilisator während der Herstellung, Lagerung und Prüfung bei der Verwendung von Testkitenzymen, wobei die Lagerungsmöglichkeit verlängert und die Effizienz der enzymatischen Testkits für diagnostische, biologische und industrielle Zwecke erhöht wird.

11. Verwendung von 1,1'-Diglycerol-phosphat nach Anspruch 1 zur Stabilisierung von Enzymen oder anderen Proteinen während der Routineverwendung für klinische, biologische und industrielle Zwecke, nämlich bei Verwendung in Biosensoren.

12. Verwendung von 1,1'-Diglycerol-phosphat nach Anspruch 1 zum Schutz von Zellmembranen, Liposomen, Liposomen enthaltenden Kosmetika oder lipidähnlichen Substanzen bei Belastungen wie Trocknung und Lyophilisierung.

13. Verwendung von 1,1'-Diglycerol-phosphat nach Anspruch 1 als Additiv für Kosmetika.

14. Verwendung von 1,1'-Diglycerol-phosphat nach Anspruch 1 zum Schutz von mikrobiellen Zellen gegen Schäden durch Lyophilisierung, Trocknung, hohe Temperaturen und Einfrieren.

## Revendications

1. Utilisation de 1,1'-di-glycérol phosphate, **caractérisée par le fait que** le composé se présente sous l'une de ses formes stéréoisomériques possibles, spécialement sous forme racémique, méso et/ou optiquement active, en tant que stabilisateur de composants cellulaires ou de cellules sous stress.

2. Procédé de synthèse chimique du 1,1'-di-glycérol phosphate représenté sur la figure 1, **caractérisé par le fait que** le composé est synthétisé en trois étapes à partir de glycérol disponible dans le commerce. La première étape consiste en une préparation de 1,2-O-cyclohexylidène-glycérol. A la deuxième étape, on prépare un sel triethylammonium de 1,1'-bis(2,3-O-cyclohexylidène-glycérol phosphate par réaction de 1,2-O-cyclohexylidène-glycérol avec du phosphoroyle chloride dans du tétrahydrofurane anhydre. A la troisième étape, le 1,1'-di-glycérol phosphate est synthétisé par le traitement du sel triethylammonium de 1,1'-bis(2,3-O-cyclohexylidène-glycérol)phosphate avec une résine échangeuse d'ions Dowex 50W-X8 ion activée.

3. Procédé de synthèse chimique du (S,S)-1,1'-di-glycérol phosphate optiquement pur, **caractérisé par le fait que** le composé est synthétisé à partir de D-mannitol disponible dans le commerce. Aux deux premières étapes, on obtient du (S)-1,2-O-isopropylidène-glycérol par réduction de l'aldéhyde correspondant. A la troisième et à la quatrième étape, le (S)-1,2-O-isopropylidène-glycérol est converti, par l'intermédiation de sel triethylammonium de (S,S)-1,1'-bis(2,3-O-isopropylidene-glycérol) phosphate en (S,S)-1,1'-di glycérol phosphate, conformément à la revendication 2.

4. Procédé de synthèse chimique du (R,R)-1,1'-di-glycérol phosphate optiquement pur, **caractérisé par** la synthétisation du composé à partir de D-mannitol disponible dans le commerce. Aux deux premières étapes, on obtient du (R)-1,2-O-isopropylidène-glycérol par réduction de l'aldéhyde correspondant. A la troisième et à la quatrième étape, le (R)-1,2-O-isopropylidène-glycérol est converti, par l'intermédiation de sel triethylammonium (R,R)-1,1'-bis(2,3-O-isopropylidène-glycérol)phosphate, en glycérol (R,R)-1,1'-di phosphate, conformément à la revendication 2.

5. Procédé de synthèse chimique du 1,1'-di-glycérol phosphate conformément aux revendications 2, 3 et 4 , **caractérisé par le fait que** le composé se présente sous l'une de ses formes stéréoisomériques possibles, spécifiquement sous ses formes racémiques, méso et/ou actives optiquement.

6. Utilisation de 1,1'-di-glycérol phosphate conformément à la revendication 1, **caractérisée par** son action en tant qu'agent thermo stabilisant pour lactate déhydrogénase, alcool déhydrogénase, rubrédoxine, et toute autre enzyme provenant de sources microbiennes, végétales ou animales, dans un but d'amélioration de leur stabilité thermique.

7. Utilisation de 1,1'-di-glycérol phosphate conformément à la revendication 1, **caractérisée par** le but de protéger enzymes ou autres protéines de la dénaturation de température causée par la purification, le transport et/ou le stockage.

8. Utilisation de 1,1'-di-glycérol phosphate conformément à la revendication 1, **caractérisée par** la protection de l'activité des enzymes dans la réaction en chaîne par polymérase (PCR) à des fins cliniques, biologiques et industrielles pendant le stockage, aussi bien que par le recyclage à haute température des enzymes.

9. Utilisation de 1,1'-di-glycérol phosphate conformément à la revendication 1, **caractérisée par** la stabilisation d'enzymes ou autres protéines pendant la lyophilisation, la dessiccation ou la lyophilisation et le stockage à basses températures.

10. Utilisation de 1,1'-di-glycérol phosphate conformément à la revendication 1, **caractérisée par** une action d'agent stabilisant pendant la fabrication, le stockage et les essais avec utilisation d'un test kit d'enzymes, et en obtenant ainsi un stockage prolongé et une efficience plus élevée des tests kits enzymatiques susmentionnés à fins diagnostiques, biologiques et industrielles.

11. Utilisation de 1,1'-di-glycérol phosphate conformément à la revendication 1, **caractérisée par** la stabilisation des enzymes ou autres protéines pendant leur utilisation de routine à des fins cliniques, biologiques et industrielles, à savoir quand elles sont utilisées dans les biocapteurs.

12. Utilisation de 1,1'-di-glycérol phosphate conformément à la revendication 1, **caractérisée par** une protection contre des stress tels que la dessiccation et la lyophilisation de membranes cellulaires, de liposomes, de produits cosmétiques contenant des liposomes ou de substances apparentées aux lipides.

13. Utilisation de 1,1'-di-glycérol phosphate conformément à la revendication 1, **caractérisée par** leur addition à des produits cosmétiques.

14. Utilisation de 1,1'-di-glycérol phosphate conformément à la revendication 1, **caractérisée par** la protection contre les dommages causés aux cellules microbiennes par la lyophilisation, la dessiccation, les hautes températures et la congélation.
